(19) **Europäisches Patentamt / European Patent Office / Office européen des brevets**

(11) **EP 3 834 949 A1**

(12) **EUROPEAN PATENT APPLICATION**

(43) Date of publication:
16.06.2021 Bulletin 2021/24

(51) Int Cl.:
B05B 17/06 (2006.01)   A24F 40/05 (2020.01)
B06B 1/02 (2006.01)    A61M 15/00 (2006.01)
A61M 11/00 (2006.01)   A61M 16/00 (2006.01)

(21) Application number: 20168245.7

(22) Date of filing: 06.04.2020

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
KH MA MD TN

(30) Priority: 15.12.2019 PCT/IB2019/060811

(71) Applicant: Shaheen Innovations Holding Limited Abu Dhabi (AE)

(72) Inventors:
• ALSHAIBA SALEH GHANNAM ALMAZROUEI, Mohammed
Abu Dhabi (AE)
• LAHOUD, Imad
Abu Dhabi (AE)

(74) Representative: Forresters IP LLP
Skygarden
Erika-Mann-Straße 11
80636 München (DE)

(54) **ULTRASONIC SYSTEMS AND METHODS**

(57)    An ultrasonic system (100) comprising: an ultrasonic transducer (5); and a frequency control module which is coupled to the ultrasonic transducer (5), wherein the frequency control module is configured to control the ultrasonic transducer (5) to oscillate at a plurality of frequencies within a predetermined sweep frequency range and to select a drive frequency for the ultrasonic transducer (5) which is between a first predetermined frequency and a second predetermined frequency to optimize the oscillation of the ultrasonic transducer (5).

FIG. 8

## Description

### FIELD

The present invention relates to ultrasonic systems and methods. The present invention more particularly relates to ultrasonic mist inhaler systems and methods for atomizing a liquid by ultrasonic vibrations.

### BACKGROUND

Electronic vaporizing inhalers are becoming popular among smokers who also want to avoid the tar and other harsh chemicals associated with traditional cigarettes and who wish to satisfy the craving for nicotine. Electronic vaporizing inhalers may contain liquid nicotine, which is typically a mixture of nicotine oil, a solvent, water, and often flavoring. When the user draws, or inhales, on the electronic vaporizing inhaler, the liquid nicotine is drawn into a vaporizer where it is heated into a vapor. As the user draws on the electronic vaporizing inhaler, the vapor containing the nicotine is inhaled. Such electronic vaporizing inhalers may have medical purpose.

Electronic vaporizing inhalers and other vapor inhalers typically have similar designs. Most electronic vaporizing inhalers feature a liquid nicotine reservoir with an interior membrane, such as a capillary element, typically cotton, that holds the liquid nicotine so as to prevent leaking from the reservoir. Nevertheless, these cigarettes are still prone to leaking because there is no obstacle to prevent the liquid from flowing out of the membrane and into the mouthpiece. A leaking electronic vaporizing inhaler is problematic for several reasons. As a first disadvantage, the liquid can leak into the electronic components, which can cause serious damage to the device. As a second disadvantage, the liquid can leak into the electronic vaporizing inhaler mouthpiece, and the user may inhale the unvaporized liquid.

Electronic vaporizing inhalers are also known for providing inconsistent doses between draws. The aforementioned leaking is one cause of inconsistent doses because the membrane may be oversaturated or undersaturated near the vaporizer. If the membrane is oversaturated, then the user may experience a stronger than desired dose of vapor, and if the membrane is undersaturated, then the user may experience a weaker than desired dose of vapor. Additionally, small changes in the strength of the user's draw may provide stronger or weaker doses. Inconsistent dosing, along with leaking, can lead to faster consumption of the vaping liquid.

Additionally, conventional electronic vaporizing inhalers tend to rely on inducing high temperatures of a metal heating component configured to heat a liquid in the e-cigarette, thus vaporizing the liquid that can be breathed in. Problems with conventional electronic vaporizing inhalers may include the possibility of burning metal and subsequent breathing in of the metal along with the burnt liquid. In addition, some may not prefer the burnt smell caused by the heated liquid.

Thus, a need exists in the art for improved ultrasonic systems and methods which seek to address at least some of the problems described herein.

### BRIEF SUMMARY

According to one aspect of the present invention, there is provided an ultrasonic system comprising: an ultrasonic transducer; and a frequency control module which is coupled to the ultrasonic transducer, wherein the frequency control module is configured to control the ultrasonic transducer to oscillate at a plurality of frequencies within a predetermined sweep frequency range and to select a drive frequency for the ultrasonic transducer which is between a first predetermined frequency and a second predetermined frequency to optimize the oscillation of the ultrasonic transducer.

In some embodiments, the frequency control module is configured to control the ultrasonic transducer to oscillate at a plurality of frequencies which track progressively across the predetermined sweep frequency range.

In some embodiments, the system further comprises: an Analog-to-Digital converter which is coupled to the ultrasonic transducer and to the frequency control module, wherein the frequency control module is configured to monitor an Analog-to-Digital Conversion value of the Analog-to-Digital converter as the frequency control module controls the ultrasonic transducer to oscillate at the plurality of frequencies within the predetermined sweep frequency range.

In some embodiments, the Analog-to-Digital Conversion value is a parameter of the Analog-to-Digital converter which is proportional to a voltage across the ultrasonic transducer.

In some embodiments, the Analog-to-Digital Conversion value is a parameter of the Analog-to-Digital converter which is proportional to a current flowing through the ultrasonic transducer.

In some embodiments, the frequency control module is configured to detect when the Analog-to-Digital Conversion value is above a predetermined threshold and to lock the drive frequency of the ultrasonic transducer when the Analog-to-Digital Conversion value is above the predetermined threshold.

In some embodiments, the frequency control module is configured to control the ultrasonic transducer to oscillate at a plurality of frequencies within the predetermined sweep frequency range periodically during the operation of the system.

In some embodiments, the first predetermined frequency is 2.8 MHz and the second predetermined frequency is 3.2 MHz.

According to another aspect of the present invention, there is provided ultrasonic mist inhaler device comprising the system of any one of claims 1-8 as defined hereinafter, wherein the device further comprises: a liquid reservoir structure comprising a liquid chamber adapted to receive liquid to be atomized, a sonication chamber in fluid communication with the liquid chamber, wherein the ultrasonic transducer is configured to oscillate liquid within the sonication chamber.

According to a further aspect of the present invention, there is provided method for controlling the oscillation of an ultrasonic transducer, the method comprising: applying a drive signal to control an ultrasonic transducer to oscillate at a plurality of frequencies within a predetermined sweep frequency range; and selecting a drive frequency for the ultrasonic transducer which is between a first predetermined frequency and a second predetermined frequency to optimize the oscillation of the ultrasonic transducer.

In some embodiments, the method further comprises: applying a drive signal to control the ultrasonic transducer to oscillate at a plurality of frequencies which track progressively across the predetermined sweep frequency range.

In some embodiments, method further comprises: monitoring an Analog-to-Digital Conversion value of the Analog-to-Digital converter as the ultrasonic transducer oscillates at the plurality of frequencies within the predetermined sweep frequency range.

In some embodiments, the method further comprises: detecting when the Analog-to-Digital Conversion value is above a predetermined threshold; and locking the drive frequency of the ultrasonic transducer when the Analog-to-Digital Conversion value is above the predetermined threshold.

In some embodiments, the method further comprises: controlling the ultrasonic transducer to oscillate at a plurality of frequencies within the predetermined sweep frequency range periodically.

In some embodiments, the first predetermined frequency is 2.8 MHz and the second predetermined frequency is 3.2 MHz.

The expression "means of ultrasonic vibrations" is similar to the expression "ultrasonic oscillation component" used in the patent application PCT/IB2019/055192.

It is noted that the expression "mist" used in the invention means the liquid is not heated as usually in traditional inhalers known from the prior art. In fact, traditional inhalers use heating elements to heat the liquid above its boiling temperature to produce a vapor, which is different from a mist.

In fact, when sonicating liquids at high intensities, the sound waves that propagate into the liquid media result in alternating high-pressure (compression) and low-pressure (rarefaction) cycles, at different rates depending on the frequency. During the low-pressure cycle, high-intensity ultrasonic waves create small vacuum bubbles or voids in the liquid. When the bubbles attain a volume at which they can no longer absorb energy, they collapse violently during a high-pressure cycle. This phenomenon is termed cavitation. During the implosion, very high pressures are reached locally. At cavitation, broken capillary waves are generated, and tiny droplets break the surface tension of the liquid and are quickly released into the air, taking mist form.

The following will explain more precisely the cavitation phenomenon.

When the liquid is atomized by ultrasonic vibrations, micro water bubbles are produced in the liquid.

The bubble production is a process of formation of cavities created by the negative pressure generated by intense ultrasonic waves generated by the means of ultrasonic vibrations.

High intensity ultrasonic sound waves leading to rapid growth of cavities with relatively low and negligible reduction in cavity size during the positive pressure cycle.

Ultrasound waves, like all sound waves, consist of cycles of compression and expansion. When in contact with a liquid, Compression cycles exert a positive pressure on the liquid, pushing the molecules together. Expansion cycles exert a negative pressure, pulling the molecules away from another.

Intense ultrasound waves create regions of positive pressure and negative pressure. A cavity can form and grow during the episodes of negative pressure. When the cavity attains a critical size, the cavity implodes.

The amount of negative pressure needed depends on the type and purity of the liquid. For truly pure liquids, tensile strengths are so great that available ultrasound generators cannot produce enough negative pressure to make cavities. In pure water, for instance, more than 1,000 atmospheres of negative pressure would be required, yet the most powerful ultrasound generators produce only about 50 atmospheres of negative pressure. The tensile strength of liquids is reduced by the gas trapped within the crevices of the liquid particles. The effect is analogous to the reduction in strength that occurs from cracks in solid materials. When a crevice filled with gas is exposed to a negative-pressure cycle from a sound wave, the reduced pressure makes the gas in the crevice expand until a small bubble is released into solution.

However, a bubble irradiated with ultrasound continually absorbs energy from alternating compression and expansion cycles of the sound wave. These cause the bubbles to grow and contract, striking a dynamic balance between the void inside the bubble and the liquid outside. In some cases, ultrasonic waves will sustain a bubble that simply oscillates in size. In other cases, the average size of the bubble will increase.

Cavity growth depends on the intensity of sound. High-intensity ultrasound can expand the cavity so rapidly during the negative-pressure cycle that the cavity never has a chance to shrink during the positive-pressure cycle. In this process, cavities can grow rapidly in the course of a single cycle of sound.

For low-intensity ultrasound the size of the cavity oscillates in phase with the expansion and compression cycles. The surface of a cavity produced by low-intensity ultrasound is slightly greater during expansion cycles than during compression cycles. Since the amount of gas that diffuses in or out of the cavity depends on the surface area, diffusion into the cavity during expansion cycles will be slightly greater than diffusion out during compression cycles. For each cycle of sound, then, the cavity expands a little more than it shrinks. Over many cycles the cavities will grow slowly.

It has been noticed that the growing cavity can eventually reach a critical size where it will most efficiently absorb energy from the ultrasound. The critical size depends on the frequency of the ultrasound wave. Once a cavity has experienced a very rapid growth caused by high intensity ultrasound, it can no longer absorb energy as efficiently from the sound waves. Without this energy input the cavity can no longer sustain itself. The liquid rushes in and the cavity implodes due to a non-linear response.

The energy released from the implosion causes the liquid to be fragmented into microscopic particles which are dispersed into the air as mist.

The equation for description of the above non-linear response phenomenon may be described by the "Rayleigh-Plesset" equation. This equation can be derived from the "Navier-Stokes" equation used in fluid dynamics.

The inventors approach was to rewrite the "Rayleigh-Plesset" equation in which the bubble volume, V, is used as the dynamic parameter and where the physics describing the dissipation is identical to that used in the more classical form where the radius is the dynamic parameter.

The equation used derived as follows:

$$\frac{\left|\frac{1}{c^2}\frac{\delta^2\phi}{\delta t^2}\right|}{\nabla^2\phi} \sim \left(\frac{R}{\lambda}\right)^2 \ll 1$$

$$\frac{1}{4\pi}\left(\frac{4\pi}{3V}\right)^{\frac{1}{3}}\left(\ddot{V} - \frac{\dot{V}^2(t)}{6V}\right) = \frac{1}{\rho_0}\left(\left(p_0 + 2\sigma\left(\frac{4\pi}{3V_0}\right)^{\frac{1}{3}} - p_V\right)\left(\frac{V_0}{V}\right)^{\kappa} + p_V - 2\sigma\left(\frac{4\pi}{3V}\right)^{\frac{1}{3}} - p_0 - P(t)\right)$$

wherein:

V is the bubble volume

$V_0$ is the equilibrium bubble volume

$\rho_0$ is the liquid density (assumed to be constant)

$\sigma$ is the surface tension

$p_V$ is the vapor pressure

$p_0$ is the static pressure in the liquid just outside the bubble wall

$\kappa$ is the polytropic index of the gas

$t$ is the time

$R(t)$ is the bubble radius

P(t) is the applied pressure

$c$ is the speed sound of the liquid

*Φ* is the velocity potential

*λ* is the wavelength of the insonifying field

In the ultrasonic mist inhaler, the liquid has a kinematic viscosity between 1.05 Pa.sec and 1.412 Pa.sec.

By solving the above equation with the right parameters of viscosity, density and having a desired target bubble volume of liquid spray into the air, it has been found that the frequency range of 2.8MHz to 3.2MHz for liquid viscosity range of 1.05 Pa.s and 1.412 Pa.s produce a bubble volume of about 0.25 to 0.5 microns

The process of ultrasonic cavitation has a significant impact on the nicotine concentration in the produced mist.

No heating elements are involved, thereby leading to no burnt elements and reducing second-hand smoke effects.

Preferably, said liquid comprises 57-70 % (w/w) vegetable glycerin and 30-43% (w/w) propylene glycol, said propylene glycol including nicotine and preferably flavorings.

In the ultrasonic mist inhaler, a capillary element may extend between the sonication chamber and the liquid chamber.

In the ultrasonic mist inhaler, the capillary element is a material at least partly in bamboo fibers.

The capillary element according to the invention allows a high absorption capacity, a high rate of absorption as well as a high fluid-retention ratio.

It was found that the inherent properties of the proposed material used for the capillarity have a significant impact on the efficient functioning of the ultrasonic mist inhaler.

Further, inherent properties of the proposed material include a good hygroscopicity while maintaining a good permeability. This allows the drawn liquid to efficiently permeate the capillary while the observed high absorption capacity allows the retention of a considerable amount of liquid thus allowing the ultrasonic mist inhaler to last for a longer time when compared with the other products available in the market.

Another significant advantage of using the bamboo fibers is the naturally occurring antimicrobial bio-agent namely "Kun" inherently present within the bamboo fiber making it antibacterial, anti-fungal and odor resistant, making it suitable for medical applications.

The inherent properties have been verified using numerical analysis regarding the benefits of the bamboo fiber for sonication.

The following formulae have been tested with bamboo fibers material and others material such a cotton, paper, or other fiber strands for the use as capillary element and demonstrates that bamboo fibers have much better properties for the use in sonication:

$$C = A + \frac{T}{W_f} - \frac{1}{P_f} + (1 - \alpha)\frac{V_d}{W_f}$$

wherein:

*C (cc/gm of fluid/gm)* is the volume per mass of the liquid absorbed divided by the dry mass of the capillary element,

*A ($cm^2$)* is the total surface area of the capillary element

*T (cm)* is the thickness of the capillary element,

$W_f$ *(gm)* is the mass of the dry capillary element,

$P_f$ *(cc/g.sec)* is the density of the dry capillary element,

*α* is the ratio of increase in volume of capillary element upon wetting to the volume of liquid diffused in the capillary element,

$V_d$ *(cc)* is the amount of liquid diffused in the capillary element,

$$Absorbent\ Rate, Q = \frac{\pi r \gamma 1 \cos\theta}{2\eta} \cdot \left(\frac{T}{W_f} - \frac{1}{AP_f}\right)$$

$Q$ (*cc/sec*) is the amount of liquid absorbed per unit time,

$r$ (*cm*) is the radius of the pores within the capillary element,

$\gamma$ (*N/m*) is the surface tension of the liquid,

$\theta$ (*degrees*) is the angle of contact of the fiber,

$\eta$ (*m²/sec*) is the viscosity of the fluid.

In the ultrasonic mist inhaler, the capillary element may be a material at least partly in bamboo fibers.

In the ultrasonic mist inhaler, the capillary element material may be 100% bamboo fiber.

Extensive testing have concluded that a 100% pure bamboo fiber is the most optimal choice for sonication.

In the ultrasonic mist inhaler, the capillary element material may be at least 75% bamboo fiber and, preferably, 25% cotton.

Capillary element from 100% pure bamboo fiber or with a high percentage of bamboo fibers demonstrates a high absorption capacity as well as improved fluid transmission making it an optimal choice for the application of the ultrasonic mist inhaler.

In the ultrasonic mist inhaler, the capillary element may have a flat shape.

In the ultrasonic mist inhaler, the capillary element may comprise a central portion and a peripheral portion.

In the ultrasonic mist inhaler, the peripheral portion may have an L-shape cross section extending down to the liquid chamber.

In the ultrasonic mist inhaler, the central portion may have a U-shape cross section extending down to the sonication chamber.

The ultrasonic mist inhaler according to the invention, wherein said liquid to be received in the liquid chamber comprises 57-70 % (w/w) vegetable glycerin and 30-43% (w/w) propylene glycol, said propylene glycol including nicotine and flavorings.

An ultrasonic mist inhaler or a personal ultrasonic atomizer device, comprising:

- a liquid reservoir structure comprising a liquid chamber or cartridge adapted to receive liquid to be atomized,

- a sonication chamber in fluid communication with the liquid chamber or cartridge,

wherein said liquid to be received in the liquid chamber comprises 57-70 % (w/w) vegetable glycerin and 30-43% (w/w) propylene glycol, said propylene glycol including nicotine and flavorings.

**BRIEF DESCRIPTION OF THE DRAWINGS**

Some embodiments are illustrated by way of example and not limitation in the figures of the accompanying drawings.

FIG. 1 is an exploded view of components of the ultrasonic mist inhaler according to an embodiment of the invention.

FIG. 2 is an exploded view of components of the inhaler liquid reservoir structure according to an embodiment of the invention.

FIG. 3 is a cross section view of components of the inhaler liquid reservoir structure according to FIG. 1.

FIG. 4A is an isometric view of an airflow member of the inhaler liquid reservoir structure according to FIGs. 2 and 3.

FIG. 4B is a cross section view of the airflow member shown in FIG. 4A.

FIG. 5 is schematic diagram showing a piezoelectric transducer modelled as an RLC circuit.

FIG. 6 is graph of frequency versus log impedance of an RLC circuit.

FIG. 7 is graph of frequency versus log impedance showing inductive and capacitive regions of operation of a piezoelectric transducer.

FIG. 8 is flow diagram showing the operation of a frequency control module of some embodiments.

**DETAILED DESCRIPTION**

The foregoing summary, as well as the following detailed description of certain embodiments of the present invention, will be better understood when read in conjunction with the appended drawings.

As used herein, an element recited in the singular and preceded with the word "a" or "an" should be understood as not excluding plural of said elements, unless such exclusion is explicitly stated. Furthermore, the references to "one embodiment" of the present invention are not intended to be interpreted as excluding the existence of additional embodiments that also incorporate the recited features. Moreover, unless explicitly stated to the contrary, embodiments "comprising" or "having" an element or a plurality of elements having a particular property may include additional such elements not having that property.

In some embodiments, the present invention is directed to an ultrasonic mist inhaler. The present description and accompanying figures are directed to the electronic vaporizing inhaler embodiment. However, other embodiments are envisioned, such as an inhaler for hookah, flavored liquids, medicine, and herbal supplements. Additionally, the device can be packaged to look like an object other than a cigarette. For instance, the device could resemble another smoking instrument, such as a pipe, water pipe, or slide, or the device could resemble another non-smoking related object.

Ultrasonic mist inhalers are either disposable or reusable. The term "reusable" as used herein implies that the energy storage device is rechargeable or replaceable or that the liquid is able to be replenished either through refilling or through replacement of the liquid reservoir structure. Alternatively, in some embodiments reusable electronic device is both rechargeable and the liquid can be replenished. A disposable embodiment will be described first, followed by a description of a reusable embodiment.

Conventional electronic vaporizing inhaler tend to rely on inducing high temperatures of a metal component configured to heat a liquid in the inhaler, thus vaporizing the liquid that can be breathed in. The liquid typically contains nicotine and flavorings blended into a solution of propylene glycol (PG) and vegetable glycerin (VG), which is vaporized via a heating component at high temperatures. Problems with conventional inhaler may include the possibility of burning metal and subsequent breathing in of the metal along with the burnt liquid. In addition, some may not prefer the burnt smell or taste caused by the heated liquid.

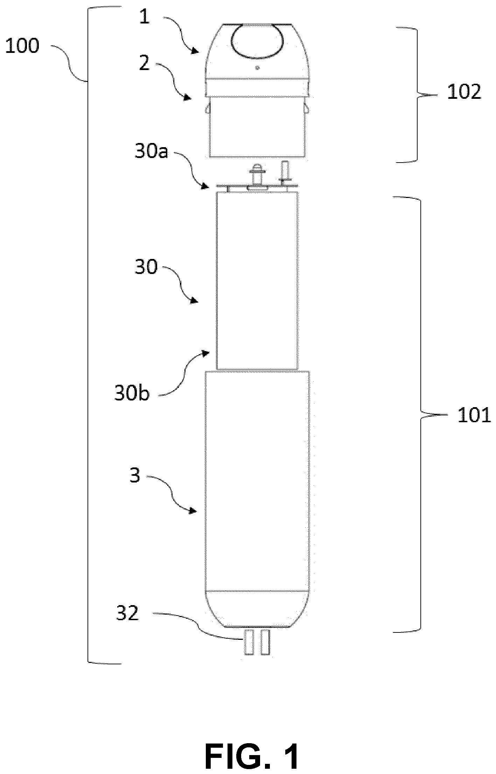

FIG. 1 to FIG. 4 illustrates an embodiment of an ultrasonic inhaler comprising a sonication chamber.

FIG. 1 depicts a disposable ultrasonic mist inhaler embodiment 100. As can be seen in FIG. 1, the ultrasonic mist inhaler 100 has a cylindrical body with a relatively long length as compared to the diameter. In terms of shape and appearance, the ultrasonic mist inhaler 100 is designed to mimic the look of a typical cigarette. For instance, the inhaler can feature a first portion 101 that primarily simulates the tobacco rod portion of a cigarette and a second portion 102 that primarily simulates a filter. In the disposable embodiment of the invented device, the first portion and second portion are regions of a single, but-separable device. The designation of a first portion 101 and a second portion 102 is used to conveniently differentiate the components that are primarily contained in each portion.

As can be seen in FIG. 1, the ultrasonic mist inhaler comprises a mouthpiece 1, a liquid reservoir structure 2 and a casing 3. The first portion 101 comprises the casing 3 and the second portion 102 comprises the mouthpiece 1 and the reservoir structure 2.

The first portion 101 contains the power supply energy.

An electrical storage device 30 powers the ultrasonic mist inhaler 100. The electrical storage device 30 can be a battery, including but not limited to a lithium-ion, alkaline, zinc-carbon, nickel-metal hydride, or nickel-cadmium battery; a super capacitor; or a combination thereof. In the disposable embodiment, the electrical storage device 30 is not rechargeable, but, in the reusable embodiment, the electrical storage device 30 would be selected for its ability to recharge. In the disposable embodiment, the electrical storage device 30 is primarily selected to deliver a constant voltage over the life of the inhaler 100. Otherwise, the performance of the inhaler would degrade over time. Preferred electrical storage devices that are able to provide a consistent voltage output over the life of the device include lithium-ion and lithium polymer batteries.

The electrical storage device 30 has a first end 30a that generally corresponds to a positive terminal and a second end 30b that generally corresponds to a negative terminal. The negative terminal is extending to the first end 30a.

Because the electrical storage device 30 is located in the first portion 101 and the liquid reservoir structure 2 is located in the second portion 102, the joint needs to provide electrical communication between those components. In the present invention, electrical communication is established using at least an electrode or probe that is compressed together when the first portion 101 is tightened into the second portion 102.

In order for this embodiment to be reusable, the electrical storage device 30 is rechargeable. The casing 3 contains a charging port 32.

The integrated circuit 4 has a proximal end 4a and a distal end 4b. The positive terminal at the first end 30a of the electrical storage device 30 is in electrical communication with a positive lead of the flexible integrated circuit 4. The negative terminal at the second end 30b of the electrical storage device 30 is in electrical communication with a negative lead of the integrated circuit 4. The distal end 4b of the integrated circuit 4 comprise a microprocessor. The microprocessor is configured to process data from a sensor, to control a light, to direct current flow to means of ultrasonic vibrations 5 in the second portion 102, and to terminate current flow after a preprogrammed amount of time.

The sensor detects when the ultrasonic mist inhaler 100 is in use (when the user draws on the inhaler) and activates the microprocessor. The sensor can be selected to detect changes in pressure, air flow, or vibration. In a preferred embodiment, the sensor is a pressure sensor. In the digital embodiment, the sensor takes continuous readings which in turn requires the digital sensor to continuously draw current, but the amount is small and overall battery life would be negligibly affected.

In some embodiments, the integrated circuit 4 comprises a H bridge, preferably formed by 4 MOSFETs to convert a direct current into an alternate current at high frequency.

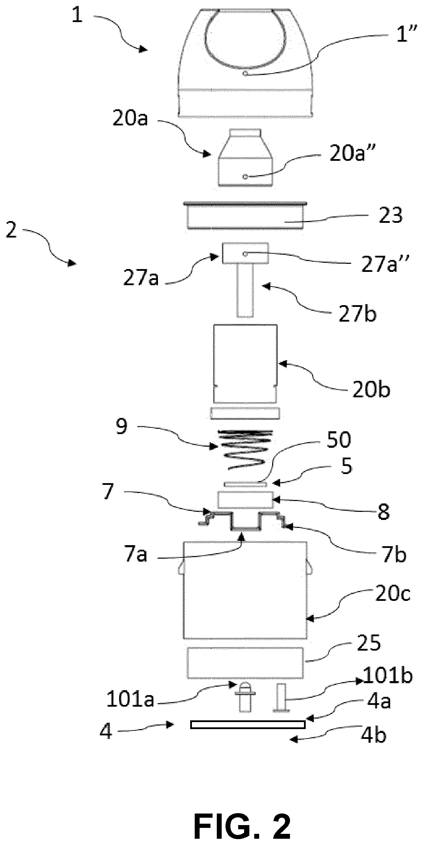

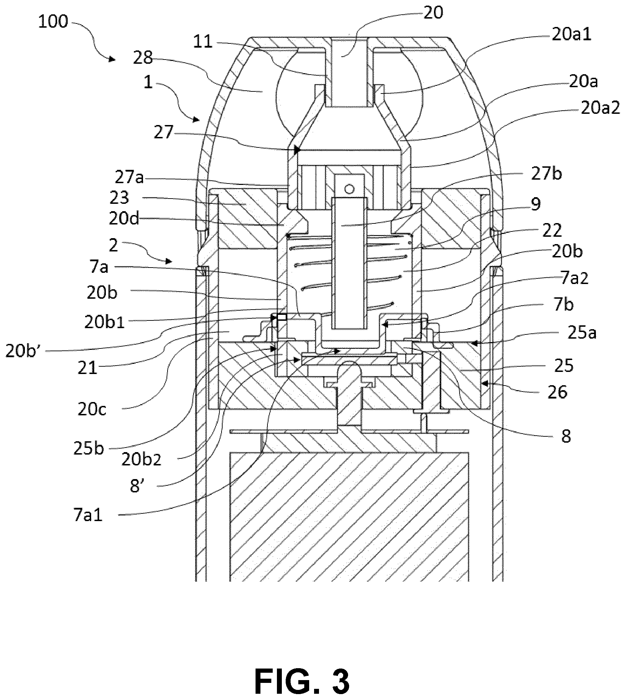

Referring to FIG. 2 and FIG. 3, illustrations of a liquid reservoir structure 2 according to an embodiment are shown. The liquid reservoir structure 2 comprises a liquid chamber 21 adapted to receive liquid to be atomized and a sonication chamber 22 in fluid communication with the liquid chamber 21.

In the embodiment shown, the liquid reservoir structure 2 comprises an inhalation channel 20 providing an air passage from the sonication chamber 22 toward the surroundings.

As an example of sensor position, the sensor may be located in the sonication chamber 22.

The inhalation channel 20 has a frustoconical element 20a and an inner container 20b.

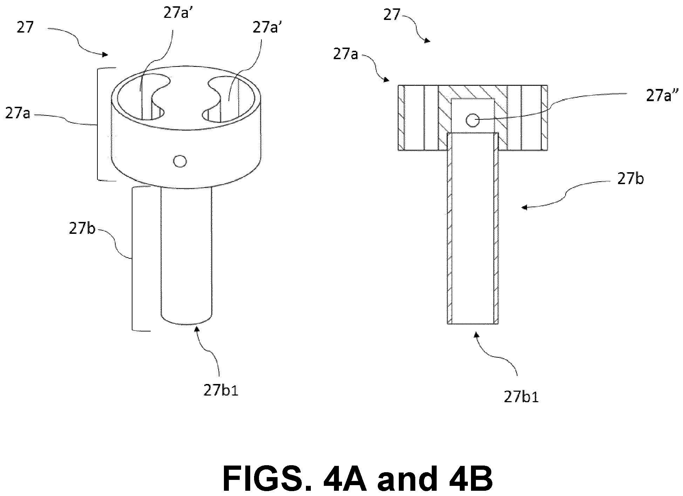

As depicted in FIGs. 4A and 4B, further the inhalation channel 20 has an airflow member 27 for providing air flow from the surroundings to the sonication chamber 22.

The airflow member 27 has an airflow bridge 27a and an airflow duct 27b made in one piece, the airflow bridge 27a having two airway openings 27a' forming a portion of the inhalation channel 20 and the airflow duct 27b extending in the sonication chamber 22 from the airflow bridge 27a for providing the air flow from the surroundings to the sonication chamber.

The airflow bridge 27a cooperates with the frustoconical element 20a at the second diameter 20a2.

The airflow bridge 27a has two opposite peripheral openings 27a" providing air flow to the airflow duct 27b.

The cooperation with the airflow bridge 27a and the frustoconical element 20a is arranged so that the two opposite peripheral openings 27a" cooperate with complementary openings 20a" in the frustoconical element 20a.

The mouthpiece 1 and the frustoconical element 20a are radially spaced and an airflow chamber 28 is arranged between them.

As depicted in FIG. 1 and 2, the mouthpiece 1 has two opposite peripheral openings 1".

The peripheral openings 27a", 20a", 1" of the airflow bridge 27a, the frustoconical element 20a and the mouthpiece 1 directly supply maximum air flow to the sonication chamber 22.

The frustoconical element 20a includes an internal passage, aligned in the similar direction as the inhalation channel 20, having a first diameter 20a1 less than that of a second diameter 20a2, such that the internal passage reduces in diameter over the frustoconical element 20a.

The frustoconical element 20a is positioned in alignment with the means of ultrasonic vibrations 5 and a capillary element 7, wherein the first diameter 20a1 is linked to an inner duct 11 of the mouthpiece 1 and the second diameter 20a2 is linked to the inner container 20b.

The inner container 20b has an inner wall delimiting the sonication chamber 22 and the liquid chamber 21.

The liquid reservoir structure 2 has an outer container 20c delimiting the outer wall of the liquid chamber 21.

The inner container 20b and the outer container 20c are respectively the inner wall and the outer wall of the liquid chamber 21.

The liquid reservoir structure 2 is arranged between the mouthpiece 1 and the casing 3 and is detachable from the mouthpiece 1 and the casing 3.

The liquid reservoir structure 2 and the mouthpiece 1 or the casing 3 may include complimentary arrangements for engaging with one another; further such complimentary arrangements may include one of the following: a bayonet type arrangement; a threaded engaged type arrangement; a magnetic arrangement; or a friction fit arrangement; wherein the liquid reservoir structure 2 includes a portion of the arrangement and the mouthpiece 1 or the casing 3 includes the complimentary portion of the arrangement.

In the reusable embodiment, the components are substantially the same. The differences in the reusable embodiment vis-a-vis the disposable embodiment are the accommodations made to replace the liquid reservoir structure 2.

As shown in FIG. 3, the liquid chamber 21 has a top wall 23 and a bottom wall 25 closing the inner container 20b and the outer container 20c of the liquid chamber 21.

The capillary element 7 is arranged between a first section 20b1 and a second section 20b2 of the inner container 20b.

The capillary element 7 has a flat shape extending from the sonication chamber to the liquid chamber.

As depicted in FIG. 2 or 3, the capillary element 7 comprises a central portion 7a in U-shape and a peripheral portion 7b in L-shape.

The L-shape portion 7b extends into the liquid chamber 21 on the inner container 20b and along the bottom wall 25.

The U-shape portion 7a is contained into the sonication chamber 21. The U-shape portion 7a on the inner container 20b and along the bottom wall 25.

In the ultrasonic mist inhaler, the U-shape portion 7a has an inner portion 7a1 and an outer portion 7a2, the inner portion 7a1 being in surface contact with an atomization surface 50 of the means of ultrasonic vibrations 5 and the outer portion 7a2 being not in surface contact with the means of ultrasonic vibrations 5.

The bottom wall 25 of the liquid chamber 21 is a bottom plate 25 closing the liquid chamber 21 and the sonication chamber 22. The bottom plate 25 is sealed, thus preventing leakage of liquid from the sonication chamber 22 to the casing 3.

The bottom plate 25 has an upper surface 25a having a recess 25b on which is inserted an elastic member 8. The means of ultrasonic vibrations 5 are supported by the elastic member 8. The elastic member 8 is formed from an annular plate-shaped rubber having an inner hole 8' wherein a groove is designed for maintaining the means of ultrasonic vibrations 5.

The top wall 23 of the liquid chamber 21 is a cap 23 closing the liquid chamber 23.

The top wall 23 has a top surface 23 representing the maximum level of the liquid that the liquid chamber 21 may contain and the bottom surface 25 representing the minimum level of the liquid in the liquid chamber 21.

The top wall 23 is sealed, thus preventing leakage of liquid from the liquid chamber 21 to the mouthpiece 1.

The top wall 23 and the bottom wall 25 are fixed to the liquid reservoir structure 2 by means of fixation such as screws, glue or friction.

As depicted in FIG. 3, the elastic member is in line contact with the means of ultrasonic vibrations 5 and prevents contact between the means of ultrasonic vibrations 5 and the inhaler walls, suppression of vibrations of the liquid reservoir structure are more effectively prevented. Thus, fine particles of the liquid atomized by the atomizing member can be sprayed farther.

As depicted in FIG. 3, the inner container 20b has openings 20b' between the first section 20b1 and the second section 20b2 from which the capillary element 7 is extending from the sonication chamber 21. The capillary element 7 absorbs liquid from the liquid chamber 21 through the apertures 20b'. The capillary element 7 is a wick. The capillary element 7 transports liquid to the sonication chamber 22 via capillary action. Preferably the capillary element 7 is made of bamboo fibers. Preferably, the capillary element 7 may be of a thickness between 0.27mm and 0.32mm and have a density between 38 $g/m^2$ and 48 $g/m^2$.

As can be seen in FIG. 3, the means of ultrasonic vibrations 5 are disposed directly below the capillary element 7.

The means of ultrasonic vibrations 5 may be a transducer. For example, the means of ultrasonic vibrations 5 may be a piezoelectric transducer, preferably designed in a circular plate-shape. The material of the piezoelectric transducer is preferably in ceramic.

A variety of transducer materials can also be used for the means of ultrasonic vibrations 5.

The end of the airflow duct 27b1 faces the means of ultrasonic vibrations 5. The means of ultrasonic vibrations 5 are in electrical communication with electrical contactors 101a, 101b. It is noted that, the distal end 4b of the integrated circuit 4 has an inner electrode and an outer electrode. The inner electrode contacts the first electrical contact 101a which is a spring contact probe, and the outer electrode contacts the second electrical contact 101b which is a side pin. Via the integrated circuit 4, the first electrical contact 101a is in electrical communication with the positive terminal of the electrical storage device 30 by way of the microprocessor, while the second electrical contact 101b is in electrical communication with the negative terminal of the electrical storage device 30.

The electrical contacts 101a, 101b crossed the bottom plate 25. The bottom plate 25 is designed to be received inside the perimeter wall 26 of the liquid reservoir structure 2. The bottom plate 25 rests on complementary ridges, thereby creating the liquid chamber 21 and sonication chamber 22.

The inner container 20b comprises a circular inner slot 20d on which a mechanical spring is applied.

By pushing the central portion 7a1 onto the means of ultrasonic vibrations 5, the mechanical spring 9 ensures a contact surface between them.

The liquid reservoir structure 2 and the bottom plate 25 can be made using a variety of thermoplastic materials.

When the user draws on the ultrasonic mist inhaler 100, an air flow is drawn from the peripheral openings 1" and penetrates the airflow chamber 28, passes the peripheral openings 27a" of the airflow bridge 27a and the frustoconical element 20a and flows down into the sonication chamber 22 via the airflow duct 27b directly onto the capillary element 7. At the same time, the liquid is drawn from the reservoir chamber 21 by capillarity, through the plurality of apertures 20b', and into the capillary element 7. The capillary element 7 brings the liquid into contact with the means of ultrasonic vibrations 5 of the inhaler 100. The user's draw also causes the pressure sensor to activate the integrated circuit 4, which directs current to the means of ultrasonic vibrations 5. Thus, when the user draws on the mouthpiece 1 of the inhaler 100, two actions happen at the same time. Firstly, the sensor activates the integrated circuit 4, which triggers the means of ultrasonic vibrations 5 to begin vibrating. Secondly, the draw reduces the pressure outside the reservoir chamber 21 such that flow of the liquid through the apertures 20b' begins, which saturates the capillary element 7. The capillary element 7 transports the liquid to the means of ultrasonic vibrations 5, which causes bubbles to form in a capillary channel by the means of ultrasonic vibrations 5 and mist the liquid. Then, the mist liquid is drawn by the user.

In some embodiments, the integrated circuit 4 comprises a frequency control module which is configured to control the frequency at which the means of ultrasonic vibrations 5 operates.

As described above, it is preferable for the ultrasonic mist inhaler 100 to drive the means of ultrasonic vibrations 5 with a signal having a frequency of 2.8MHz to 3.2MHz in order to vaporize a liquid having a liquid viscosity of 1.05 Pa.s to 1.412 Pa.s in order to produce a bubble volume of about 0.25 to 0.5 microns. However, for liquids with a different viscosity or for other applications it may be preferable to drive the means of ultrasonic vibrations 5 at a different frequency.

For each different application for a mist generation system, there is an optimum frequency or frequency range for driving the means of ultrasonic vibrations 5 in order to optimize the generation of mist. In embodiments where the means of ultrasonic vibrations 5 is a piezoelectric transducer, the optimum frequency or frequency range will depend on at least the following four parameters:

### 1. Transducer Manufacturing Processes

In some embodiments, the means of ultrasonic vibrations 5 comprises a piezoelectric ceramic. The piezoelectric ceramic is manufactured by mixing compounds to make a ceramic dough and this mixing process may not be consistent throughout production. This inconsistency can give rise to a range of different resonant frequencies of the cured piezoelectric ceramic.

If the resonant frequency of the piezoelectric ceramic does not correspond to the required frequency of operation of the device then no mist is produced during the operation of the device. In the case of a nicotine mist inhaler, even a slight offset in the resonant frequency of the piezoelectric ceramic is enough to impact the production of mist, meaning that the device will not deliver adequate nicotine levels to the user.

### 2. Load on transducer

During operation, any changes in the load on the piezoelectric transducer will inhibit the overall displacement of the oscillation of the piezoelectric transducer. To achieve optimal displacement of the oscillation of the piezoelectric transducer, the drive frequency must be adjusted to enable the circuit to provide adequate power for maximum displacement.

The types of loads that can affect the oscillator's efficiency can include the amount of liquid on the transducer (dampness of the wicking material), and the spring force applied to the wicking material to keep permanent contact with the transducer. It may also include the means of electrical connection.

### 3. Temperature

Ultrasonic oscillations of the piezoelectric transducer are partially damped by its assembly in a device. This may include the transducer being placed in a silicone/rubber ring, and the spring exerting pressure onto the wicking material that is above the transducer. This dampening of the oscillations causes rise in local temperatures on and around the transducer.

An increase in temperature affects the oscillation due to changes in the molecular behavior of the transducer. An increase in the temperature means more energy to the molecules of the ceramic, which temporarily affects its crystalline structure. Although the effect is reversed as the temperature reduces, a modulation in supplied frequency is required to maintain optimal oscillation. This modulation of frequency cannot be achieved with a conventional fixed frequency device.

An increase in temperature also reduces the viscosity of the solution (e-liquid) which is being vaporized, which may require an alteration to the drive frequency to induce cavitation and maintain continuous mist production. In the case of a conventional fixed frequency device, a reduction in the viscosity of the liquid without any change in the drive frequency will reduce or completely stop mist production, rendering the device inoperable.

**4. Distance to Power Source**

The oscillation frequency of the electronic circuit can change depending on the wire-lengths between the transducer and the oscillator-driver. The frequency of the electronic circuit is inversely proportional to the distance between the transducer and the remaining circuit.

Although the distance parameter is primarily fixed in a device, it can vary during the manufacturing process of the device, reducing the overall efficiency of the device. Therefore, it is desirable to modify the drive frequency of the device to compensate for the variations and optimize the efficiency of the device.

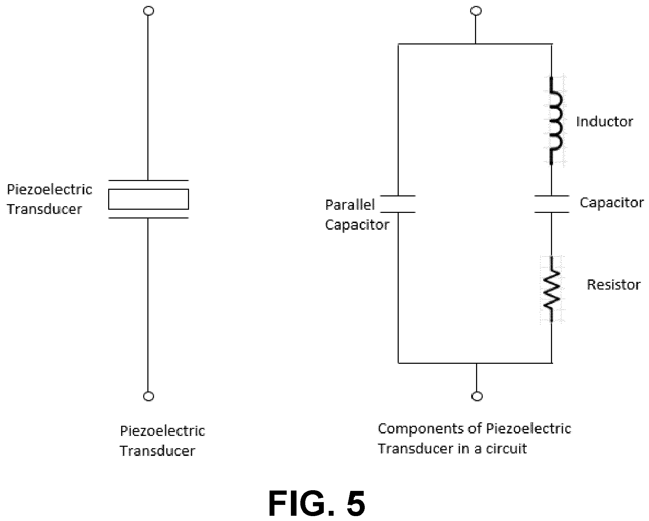

A piezoelectric transducer can be modelled as an RLC circuit in an electronic circuit, as shown in FIG. 5. The four parameters described above may be modelled as alterations to the overall inductance, capacitance, and/or resistance of the RLC circuit, changing the resonance frequency range supplied to the transducer. As the frequency of the circuit increases to around the resonance point of the transducer, the log Impedance of the overall circuit dips to a minimum and then rises to a maximum before settling to a median range.

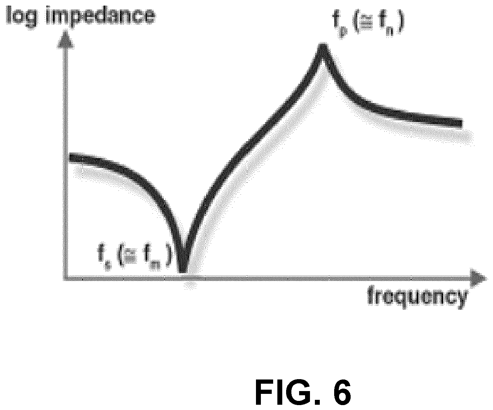

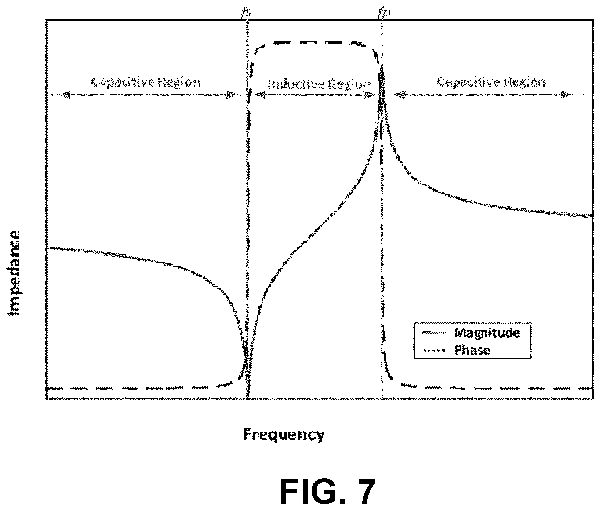

FIG. 6 shows a generic graph explaining the change in overall impedance with increase in frequency in an RLC circuit. FIG. 7 shows how a piezoelectric transducer acts as a capacitor in a first capacitive region at frequencies below a first predetermined frequency $f_s$ and in a second capacitive region at frequencies above a second predetermined frequency $f_p$. The piezoelectric transducer acts as an inductor in an inductive region at frequencies between the first and second predetermined frequencies $f_s$, $f_p$. In order to maintain optimal oscillation of the transducer and hence maximum efficiency, the current flowing through the transducer must be maintained at a frequency within the inductive region.

The frequency control module of the device of some embodiments is configured to maintain the frequency of oscillation of the piezoelectric transducer (the means of ultrasonic vibrations 5) within the inductive region, in order to maximize the efficiency of the device.

The frequency control module is configured to perform a sweep operation in which the frequency control module drives the transducer at frequencies which track progressively across a predetermined sweep frequency range. As the frequency control module performs the sweep, the frequency control module monitors an Analog-to-Digital Conversion (ADC) value of an Analog-to-Digital converter which is coupled to the transducer. In some embodiments the ADC value is a parameter of the ADC which is proportional to the voltage across the transducer. In other embodiments, the ADC value is a parameter of the ADC which is proportional to the current flowing through the transducer.

During the sweep operation, the frequency control module locates the inductive region of the frequency for the transducer. Once the frequency control module has identified the inductive region, the frequency control module records the ADC value and locks the drive frequency of the transducer at a frequency within the inductive region (i.e. between the first and second predetermined frequencies $f_s$, $f_p$) in order to optimize the ultrasonic cavitation by the transducer. When the drive frequency is locked within the inductive region, the electro-mechanical coupling factor of the transducer is maximized, thereby maximizing the efficiency of the device.

In some embodiments, the frequency control module is configured to perform the sweep operation to locate the inductive region each time the oscillation is started or re-started. In the embodiments, the frequency control module is configured to lock the drive frequency at a new frequency within the inductive region each time the oscillation is started and thereby compensate for any changes in the parameters that affect the efficiency of operation of the device.

In some embodiments, the frequency control module ensures optimal mist production and maximizes efficiency of medication delivery to the user. In some embodiments, the frequency control module optimizes the device and improves the efficiency and maximizes nicotine delivery to the user.

In other embodiments, the frequency control module optimizes the device and improves the efficiency of any other device which uses ultrasound. In some embodiments, the frequency control module is configured for use with ultrasound technology for therapeutic applications in order to extend the enhancement of drug release from an ultrasound-responsive drug delivery system. Having precise, optimal frequency during operation, ensures that the microbubbles, nanobubbles, nanodroplets, liposome, emulsions, micelles or any other delivery systems are highly effective.

In some embodiments, in order to ensure optimal mist generation and optimal delivery of compounds as described above, the frequency control module is configured to operate in a recursive mode. When the frequency control module operates in the recursive mode, the frequency control module runs the sweep of frequencies periodically during the operation of the device and monitors the ADC value to determine if the ADC value is above a predetermined threshold which is indicative of optimal oscillation of the transducer.

In some embodiments, the frequency control module runs the sweep operation while the device is in the process of aerosolizing liquid in case the frequency control module is able to identify a possible better frequency for the transducer. If the frequency control module identifies a better frequency, the frequency control module locks the drive frequency at the newly identified better frequency in order to maintain optimal operation of the device.

In some embodiments, the frequency control module runs the sweep of frequencies for a predetermined duration periodically during the operation of the device. In the case of the device of the embodiments described above, the predetermined duration of the sweep and the time period between sweeps are selected to optimize the functionality of the device. When implemented in an ultrasonic mist inhaler device, this will ensure an optimum delivery to a user throughout the user's inhalation.

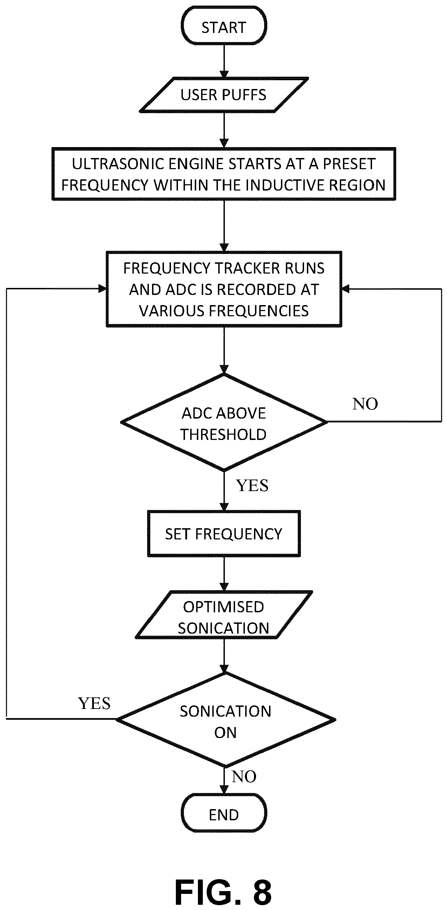

FIG. 8 shows a flow diagram of the operation of the frequency control module of some embodiments.

While the embodiments described above are predominantly directed to systems and methods for generating mist for an inhaler device, the functionality of the frequency control module can also be used for any other ultrasonic application including, but not limited to:

- Industrial sterilization: removal of microorganisms from containers with less environmental hazards.

- Fermentation: stimulating microorganisms to positively contribute to their growth, accelerating the process.

- Food industry: ultrasonic cavitation is used in extraction and emulsification of various products.

All of the above applications involving ultrasonic technology can benefit from the optimization achieved by the frequency control module which optimizes the frequency of sonication for optimal performance.

It is to be appreciated that the disclosures herein are not limited to use for nicotine delivery. Some embodiments are configured for use for various medical purposes (e.g. the delivery of CBD for pain relief, supplements for performance enhancement, albuterol/salbutamol for asthma patients, etc.)

The ultrasonic mist inhaler 100 of some embodiments is a more powerful version of current portable medical nebulizers, in the shape and size of current e-cigarettes and with a particular structure for effective vaporization. It is a healthier alternative to cigarettes and current e-cigarettes products.

The ultrasonic mist inhaler 100 of some embodiments has particular applicability for those who use electronic inhalers as a means to quit smoking and reduce their nicotine dependency. The ultrasonic mist inhaler 100 provides a way to gradually taper the dose of nicotine.

Other embodiments of the invented ultrasonic mist inhaler 100 are easily envisioned, including medicinal delivery devices.

It is to be understood that the above description is intended to be illustrative, and not restrictive. For example, the above-described embodiments may be used in combination with each other. In addition, many modifications may be made to adapt a particular situation or material to the teachings of the invention without departing from its scope.

When used in this specification and claims, the terms "comprises" and "comprising" and variations thereof mean that the specified features, steps or integers are included. The terms are not to be interpreted to exclude the presence of other features, steps or components.

**Claims**

1. An ultrasonic system comprising:

   an ultrasonic transducer; and
   a frequency control module which is coupled to the ultrasonic transducer, wherein the frequency control module is configured to control the ultrasonic transducer to oscillate at a plurality of frequencies within a predetermined sweep frequency range and to select a drive frequency for the ultrasonic transducer which is between a first predetermined frequency and a second predetermined frequency to optimize the oscillation of the ultrasonic transducer.

2. The system of claim 1, wherein the frequency control module is configured to control the ultrasonic transducer to oscillate at a plurality of frequencies which track progressively across the predetermined sweep frequency range.

3. The system of claim 1 or claim 2, wherein the system further comprises:
   an Analog-to-Digital converter which is coupled to the ultrasonic transducer and to the frequency control module, wherein the frequency control module is configured to monitor an Analog-to-Digital Conversion value of the Analog-to-Digital converter as the frequency control module controls the ultrasonic transducer to oscillate at the plurality of frequencies within the predetermined sweep frequency range.

4. The system of claim 3, wherein the Analog-to-Digital Conversion value is a parameter of the Analog-to-Digital converter which is proportional to a voltage across the ultrasonic transducer.

5. The system of claim 3, wherein the Analog-to-Digital Conversion value is a parameter of the Analog-to-Digital converter which is proportional to a current flowing through the ultrasonic transducer.

6. The system of any one of claims 3 to 5, wherein the frequency control module is configured to detect when the Analog-to-Digital Conversion value is above a predetermined threshold and to lock the drive frequency of the ultrasonic transducer when the Analog-to-Digital Conversion value is above the predetermined threshold.

7. The system of any one of the preceding claims, wherein the frequency control module is configured to control the ultrasonic transducer to oscillate at a plurality of frequencies within the predetermined sweep frequency range periodically during the operation of the system.

8. The system of any one of the preceding claims, wherein the first predetermined frequency is 2.8 MHz and the second predetermined frequency is 3.2 MHz.

9. An ultrasonic mist inhaler device comprising the system of any one of the preceding claims, wherein the device further comprises:

   a liquid reservoir structure comprising a liquid chamber adapted to receive liquid to be atomized,
   a sonication chamber in fluid communication with the liquid chamber, wherein the ultrasonic transducer is configured to oscillate liquid within the sonication chamber.

10. A method for controlling the oscillation of an ultrasonic transducer, the method comprising:

   applying a drive signal to control an ultrasonic transducer to oscillate at a plurality of frequencies within a predetermined sweep frequency range; and
   selecting a drive frequency for the ultrasonic transducer which is between a first predetermined frequency and a second predetermined frequency to optimize the oscillation of the ultrasonic transducer.

11. The method of claim 10, wherein the method further comprises:
   applying a drive signal to control the ultrasonic transducer to oscillate at a plurality of frequencies which track progressively across the predetermined sweep frequency range.

12. The method of claim 10 or claim 11, wherein the method further comprises:
   monitoring an Analog-to-Digital Conversion value of the Analog-to-Digital converter as the ultrasonic transducer oscillates at the plurality of frequencies within the predetermined sweep frequency range.

13. The method of claim 12, wherein the method further comprises:

   detecting when the Analog-to-Digital Conversion value is above a predetermined threshold; and
   locking the drive frequency of the ultrasonic transducer when the Analog-to-Digital Conversion value is above the predetermined threshold.

14. The method of any one of claims 10 to 13, wherein the method further comprises:
   controlling the ultrasonic transducer to oscillate at a plurality of frequencies within the predetermined sweep frequency range periodically.

15. The method of any one of claims 10 to 14, wherein the first predetermined frequency is 2.8 MHz and the second predetermined frequency is 3.2 MHz.

**FIG. 1**

1

1"

20a

20a"

23

2

27a

27a"

27b

20b

9

50

5

7

8

7a

7b

20c

25

101a

101b

4a

4

4b

# FIG. 2

**FIG. 3**

# FIGS. 4A and 4B

Piezoelectric Transducer

Components of Piezoelectric Transducer in a circuit

Parallel Capacitor

Inductor

Capacitor

Resistor

Piezoelectric Transducer

# FIG. 5

FIG. 6

Capacitive Region    Inductive Region    Capacitive Region

—— Magnitude
····· Phase

Impedance

Frequency

**FIG. 7**

**FIG. 8**

Europäisches
Patentamt

European
Patent Office

Office européen
des brevets

# EUROPEAN SEARCH REPORT

**Application Number**

EP 20 16 8245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/088202 A1 (DURU NICOLAS [FR]) 17 April 2008 (2008-04-17) * paragraphs [0002], [0003], [0014], [0034], [0047], [0056], [0060] - [0069]; figures 1-5 * | 1-6,9-13 | INV. B05B17/06 A24F40/05 B06B1/02 A61M15/00 A61M11/00 |
| X | US 2002/129813 A1 (LITHERLAND CRAIG [US] ET AL) 19 September 2002 (2002-09-19) * paragraphs [0001], [0041] - [0044]; figures 1-5 * | 1-7,9-14 | ADD. A61M16/00 |
| X | DE 101 22 065 A1 (PARI GMBH [DE]) 12 December 2002 (2002-12-12) * paragraphs [0014], [0026] - [0029]; figures 1-5 * | 1-6,9-13 | |
| X | WO 93/09881 A2 (MEDIX ELECTRONICS LTD [GB]) 27 May 1993 (1993-05-27) * pages 1,12, paragraphs 1,2; figures 1-13 * | 1,2, 7-11,14, 15 | |
| X | WO 2009/096346 A1 (MITSUBISHI ELECTRIC CORP [JP]; FUJIWARA SUSUMU [JP]; KOMAE SOTA [JP]) 6 August 2009 (2009-08-06) * figures 1-6 * | 1,2,7, 9-11,14 | **TECHNICAL FIELDS SEARCHED (IPC)** B05B A24F B06B A61M |
| A | US 2004/099218 A1 (YANG CHE-HUA [TW] ET AL) 27 May 2004 (2004-05-27) * paragraph [0006]; figures 1-3 * | 8,9,15 | |

The present search report has been drawn up for all claims

| Place of search | Date of completion of the search | Examiner |
|---|---|---|
| Munich | 7 September 2020 | Verger, Paul |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 8245

This annex lists the patent family members relating to the patent documents cited in  the above-mentioned European search report.
The members are as contained in the European Patent Office EDP file on
The European Patent Office is in no way liable for these particulars which are merely given for the purpose of information.

07-09-2020

| Patent document cited in search report | | Publication date | Patent family member(s) | | Publication date |
|---|---|---|---|---|---|
| US 2008088202 | A1 | 17-04-2008 | AT | 470509 T | 15-06-2010 |
| | | | EP | 1875969 A1 | 09-01-2008 |
| | | | ES | 2346581 T3 | 18-10-2010 |
| | | | FR | 2903331 A1 | 11-01-2008 |
| | | | US | 2008088202 A1 | 17-04-2008 |
| US 2002129813 | A1 | 19-09-2002 | CA | 2441190 A1 | 10-10-2002 |
| | | | EP | 1370129 A1 | 17-12-2003 |
| | | | US | 2002129813 A1 | 19-09-2002 |
| | | | WO | 02078424 A1 | 10-10-2002 |
| DE 10122065 | A1 | 12-12-2002 | NONE | | |
| WO 9309881 | A2 | 27-05-1993 | AU | 2914892 A | 15-06-1993 |
| | | | CA | 2123409 A1 | 27-05-1993 |
| | | | DE | 69220965 T2 | 05-03-1998 |
| | | | EP | 0619761 A1 | 19-10-1994 |
| | | | GB | 2265845 A | 13-10-1993 |
| | | | GB | 2291605 A | 31-01-1996 |
| | | | JP | H07506999 A | 03-08-1995 |
| | | | US | 5551416 A | 03-09-1996 |
| | | | WO | 9309881 A2 | 27-05-1993 |
| WO 2009096346 | A1 | 06-08-2009 | NONE | | |
| US 2004099218 | A1 | 27-05-2004 | DE | 10341315 A1 | 09-06-2004 |
| | | | FR | 2846894 A1 | 14-05-2004 |
| | | | GB | 2395131 A | 19-05-2004 |
| | | | JP | 2004160153 A | 10-06-2004 |
| | | | TW | 562704 B | 21-11-2003 |
| | | | US | 2004099218 A1 | 27-05-2004 |

EPO FORM P0459

For more details about this annex : see Official Journal of the European Patent Office, No. 12/82